(19) Europäisches Patentamt
European Patent Office
Office européen des brevets

(11) Publication number: **0 365 683 A1**

(12) # EUROPEAN PATENT APPLICATION published in accordance with Art. 158(3) EPC

(21) Application number: 89903794.9

(22) Date of filing: 24.03.89

(86) International application number: PCT/JP89/00313

(87) International publication number: WO 89/09052 (05.10.89 89/24)

(51) Int. Cl.5: A61K 31/19 , //A23L1/03

(30) Priority: 28.03.88 JP 75638/88
21.04.88 JP 99844/88
15.03.89 JP 164556/89

(43) Date of publication of application: 02.05.90 Bulletin 90/18

(84) Designated Contracting States: CH DE FR GB IT LI NL SE

(71) Applicant: OTSUKA PHARMACEUTICAL CO., LTD.
9, Kandatsukasacho 2-chome
Chiyoda-ku Tokyo 101(JP)

(72) Inventor: FUJII, Setsuro
323, Ebiya-cho Sanjyo-sagaru
Gokomachi-dori
Nakagyo-ku, Kyoto-shi Kyoto 604(JP)
Inventor: SHIRASAKA, Tetsuhiko
8-8-302, Uchidehama
Otsu-shi Shiga 520(JP)
Inventor: FUKUSHIMA, Masakazu
9-7, Sen-cho 2-chome
Otsu-shi Shiga 520(JP)
Inventor: SHIMAMOTO, Yuji
3-9-11, Ojigaoka
Otsu-shi Shiga 520(JP)

(74) Representative: Hansen, Bernd, Dr.rer.nat. et al
Hoffmann, Eitle & Partner Patentanwälte
Arabellastrasse 4 Postfach 81 04 20
D-8000 München 81(DE)

(54) ## COMPOSITION FOR INHIBITING ABSORPTION OF NUCLEIC ACID BASES.

(57) A composition for inhibiting absorption of nucleic acid bases in a living body, which contains as an active ingredient a lower thio alkanoic acid represented by the general formula: $R^1$-S-$R^2$-COOH (wherein $R^1$ represents a hydrogen atom or a lower alkyl group, and $R^2$ represents a lower alkylene group optionally substituted by an amino group) or its salt. This composition is effective for prophylaxis and treatment of gout in virtue of its action of inhibiting absorption of nucleic acid bases.

EP 0 365 683 A1

SPECIFICATION

# Composition for inhibiting absorption of nucleic acid base

<Technical field>

This invention relates to a composition for inhibiting absorption of nucleic acid base, more specifically relates to a composition for inhibiting absorption of nucleic acid base which is the cause of a gout.

<Background art>

An intake amount of nucleic acid base (purine form and pyrimidine form) in recent Japanese meal is gradually increased, and approaches to that of Europeans and Americans, (i.e. 600 to 1000 mg/day). As a food including a large amount of nucleic acid base, example is an animal meat, an internal, e.g. a liver or a kidney, typically a fish meat of sardine, cod, carp, flatfish, trout or the like, shellfish, bacon, beef, chicken, sausage, pork, and these soups or extracts; a grain such as oatmeal, a bean such as kidney bean or pea; a vegetable such as asparagus, mushroom, spinach.

A purine form intaked into a living body is metabolized, and a mainmetabolite, xanthine is finaly oxidized to uric acid by xanthine oxidase (purine metabolism). In this case, when the intake amount of purine form is excessive, or when an endogenous purine-metabolism abnormality is recognized, by promotion of the purine metabolism, the uric acid concentration in a blood is increased to cause a hyperuricemia, and further due to continuation of it, gout is generated by accumulation of uric acid crystal in a joint.

Accordingly, alimentary therapy using a purine restriction meal in which the content of purine is decreases is conventionally adapted to a gout patient, especially a gout patient of acute arthritis attack term or renal insufficiency complication [for example, "Rinsho eiyo"("a clinical nutrition" in Japanese), 49, 319 (1976); "Kyoto igakukai zasshi" ("Magazine of Kyoto medical associate" in Japanese), 22, 37 (1973); "Saishin Igagu" ("Recent medicine" in Japanese), 29, 486 (1974); "JAMA", 15, 7, 1096 (1955) and the like].

However, even if employing such an alimentary therapy, the decreased amount of uric acid in blood is merely about 0.5 to 1.0 mg/dl. Also, the purine restricting meal is complicated, and due to an unbalanced nutrition and a mental anguish of the patient, this therapy is far from satisfactory.

Accordingly, under the present conditions, the intake of foods containing a special large amount of nucleic acid base such as purine is merely restricted.

The main object of this invention is to provide a composition for inhibiting absorption of nucleic acid base by which an excess intake or accumulation of nucleic acid base in the living body can be restricted, during the free intake of nucleic acid base, in place of the alimentary therapy mentioned above.

<Disclosure of the invention>

As a result of research which is conducted by inventors to achieve the above object, a new fact that a composition containing, as an effective ingredient, at least one selected from thio-lower alkanoic acid or salt thereof, represented by the general formula:

$$R^1 - S - R^2 - COOH \qquad (1)$$

wherein $R^1$ is a hydrogen atom or a lower alkyl group, $R^2$ is a lower alkylene group which may have an amino group, has an excellent effect that absorption of nucleic acid base is inhibited, is found.

In the above general formula (1), example of lower alkyl group represented by $R^1$ is a linear or branched linear alkyl group having 1 to 6 of carbon atoms, such as methyl, ethyl, propyl, isopropyl, butyl, sec-butyl, tert-butyl, pentyl, hexyl or the like. Also, example of lower alkylene group which may have an amino group, represented by $R^2$, is a

linear or branched linear alkylene group having 1 to 6 of carbon atoms, which may have amino group as a substituent, such as methylene, ethylene, propylene, trimethylene, tetramethylene, pentamethylene, hexamethylene, 2-methyltetramethylene, aminomethylene, 1-aminoethylene, 2-aminotrimethylene, 1-aminotetramethylene, 2-aminopentamethylene, 1-amino-2-methyltetramethylene, 3-aminohexamethylene or the like.

As a preferred compound in the compounds shown by the general formula (1) having the above groups, for example, L-cysteine, L-methionine or the like can be employed, and in particular, L-cysteine is suitable.

The nucleic acid base in this specification includes adenine, guanine, xanthine, hypoxanthine, thymine, uracil, cytosine, or the like.

Although all compounds represented by the general formula (1) are well-known, it is not known at all that these compounds have an inhibiting action of absorption of nucleic acid base. It is also recognized that the pharmaceutically acceptable salt of the above compound (1) has the same inhibiting effect of absorption of nucleic acid base as the compound (1). As an example of such a salt, acid-addition salt obtained by adding a pharmaceutically acceptable acid to thio-lower alkanoic acid of the general formula (1) can be employed. Example of such an acid is an inorganic acid such as hydrochloric acid, sulfuric acid, phosphoric acid, hydrogen bromide; or an organic acid such as oxalic acid, maleic acid, fumaric acid, malic acid, tartaric acid, citric acid, benzoic acid, acetic acid, p-toluenesulfonic acid, ethanesulfonic acid.

In the case that thio-lower alkanoic acid of formula (1) has an acid group, a salt obtained by reacting a pharmaceutically acceptable basic compound also shows the same inhibiting action of absorption of nucleic acid base. Example of the basic compound is a metal hydroxide such as sodium hydroxide, potassium hydroxide, calcium hydroxide; an alkali metal carbonate or bicarbonate such as sodium carbonate, potassium hydrogencarbonate, sodium hydrogencarbonate; or the like.

In order to effectively inhibiting the absorption of nucleic acid base in the living body, the oral intake of the composition of this invention is desirable, since nucleic acid base is mainly absorbed in the intestinal tract.

Form for oral intake is not limited at all, and therefore may be various pharmaceutical forms as employed in the conventional medicine or general food forms. These forms can be prepared by the conventional methods, and other ingredients used together with the above ingredient can be optionally selected.

That is, the composition of this invention is prepared to the pharmaceutical form by using the effective amount of the above effective ingredient together with a pharmaceutical carrier used conventionally, e.g. a dilution agent or a vehicle such as filler, bonding agent, humidity-keeping agent, disintegrator, surface active agent, a lubricant. Typical example of the pharmaceutical form is tablet, pil, powder, liquid, suspension, emulsion, granule, capsule or the like. When forming the tablet, an example of the carrier used for manufacturing a pharmaceutical preparation is a vehicle such as lactose, sucrose, sodium chloride, glucose, urea, starch, calcium carbonate, kaolin, crystalized cellulose, silicic acid; a bonding agent such as water, ethanol, propanol, simple syrup, glucose liquid, starch liquid, gelatin solution, carboxymethyl cellulose, shellac, methyl cellulose, calcium phosphate or polyvinyl pyrrolidone; a disintegrator such as dried starch, sodium alginate, agar powder, laminaran powder, sodium hydrocarbonate, calcium carbonate, polyoxyethylene sorbitan fatty acid ester, sodium laurilsulfate, stearyl monoglyceride, starch or lactose; a desintegration inhibiting agent such as sucrose, stearin, cacao butter or hydrogenation oil; an absorption promoting agent such as quaternary ammonium base or sodium laurilsulfate; a humidity keeping agent such as glycerin or starch; an absorbent such as starch, lactose, kaolin, bentonite or colloidal silica; a lubricant such as purified talc, stearate, boric acid powder or polyethylene glycol; or the like. Furthermore, the tablet may be used in the form having a conventional coating in compliance with the necessity. Example of the coated tablet is sugar coated tablet, gelatin coated tablet, enteric coated tablet, film coated tablet, and further the tablet may be in the form of doublelayer tablet or multilayer tablet.

When forming the pil, an example of a carrier is a vehicle such as glucose, lactose, starch, cacao butter, hydrogenated vegetable oil, kaolin or talc; a bonding agent such as powdered gum arabi, powdered tragacanth, gelatin or ethanol; and a disintegrator such as laminaran or agar; or the like. Capsule is prepared by mixing the effective ingredient with the various carriers mentioned above and then filling the mixture into hard gelatin capsule, soft gelatin capsule or the like in virtue of the conventional method.

In liquid preparation such as solution, emulsion or suspension, a diluent such as water, lactic acid aqueous solution, ethyl alcohol, propylene glycol, etoxyed isostearyl alcohol, polyoxy isostearyl alcohol, polyoxyethylene sorbitane fatty acid ester or the like is used, and liquid preparation is sterilized in virtue of the conventional method. Sodium chloride, glucose, glycerin or the like can be added to the liquid preparation, and further a dissolution accelating agent, a buffer agent or the like used conventionally can be also added.

Furthermore, to various medicinal preparations mentioned above, antioxidant (e.g. butylated hydroxytoluene, propylgalate, quinone, $\alpha$-tocopherol or the like), coloring agent, preservative, perfume, flavor agent, sweetening agent or other medicine may be contained.

In the above medicinal preparations, the compound or its salt represented by general formula (1) as an effective ingredient may be contained without a special limitation, and the content of the compound or its salt is at least about 0.01% by weight, preferably about 1% to 70% by weight. In the case of tablet, it is suitable that the content of the compound or its salt is in the range of 0.01g to 1g per 1 tablet.

The effective dose of the medicinal preparation to be orally administered is decided in compliance with the medicine form, conditions such as age, sex or the like and the degree of disease of the patient to be administered. Its dose may be optionally selected without special limitation, ordinally the standard of the amount of effective ingredient is about 30mg to 150mg per 1kg of weight of patient at one day, and may be varied optionally. The administration of the preparation can be divided into 1 to 4 times per one day.

The composition of this invention can be also prepared in the food forms. Food forms are not specially limited except for orally taking, and therefore may be various eatables and drinkables ordinally well-known, such as seasoning, confectionery (Japanese confectionery, Western confectionary, ice cream, etc.), sirup, fruit processed good, vegetable processed good, animal meat processed good, fish meat processed good, delicacy, canned, bottling, refreshing drink, light quickly cooking eatable or drinkable food or the like. Such a food form is prepared by the conventional method, except that the above effective ingredient is blended in the optional step by the suitable means such as kneading, mingling, mixing, dissolving, soaking, permeating, scattering, appling, spraying, pouring or the like. The blending amount of the effective ingredient is not especially limited, and ordinally is decided so that an intake amount per one time is in the range of about 500mg to 2000mg.

In taking or administering the composition of this invention having various forms to the patient, the intake or administration time is optionally decided, and preferably is on or before and after meal, since the absorption of nucleic acid base is most active in intake of meal.

The composition of this invention may be used in the form of suppository, besides orally taking forms.

<Brief Explanation of the Drawings>

Figs. 1 to 6 show the results carried out in Examples, in order to evidence that the effective ingredient of this invention has an inhibiting action of the absorption of nucleic acid base to the living body.

<Industrial Applicability>

By taking the composition of this invention in the form suitable to restrict the absorption of nucleic acid base in the living body, excess intake or accumulation of nucleic acid base in the living body is inhibited, thereby being useful to therapy and prophylaxis of gout, etc.

<Example>

There was investigated how the effective ingredient of this invention is acted to absorption of nucleic acid base to the living body.

For this test, wistar-king rats which weighed 180g to 200g and to which bait and water were freely given were used, and were divided between control group and experimental group.

Test liquid, which was obtained by suspending 5mg/ml of each nucleic acid base (uracil, thymine, cytosine or adenine) into 5% arabic gum solution, so that the concentration of nucleic acid base is 5 mg/ml, followed by ultrasonication for 5 minutes, was orally administered to the control group. Dose of this test liquid was 1ml per 100g of rat weight, i.e. 50mg/kg. Also, another test liquid, which was obtained by adding and suspending the test compound, i.e. effective ingredient of this invention, to each suspension liquid containing 5 mg/ml of nucleic acid base, so that the concentration of the test compound is 15 mg/ml, followed by ultrasonication in accordance with the same manner as employing above, was orally administered to the experimental group. Dose of this test liquid was 1ml per 100g of rat weight, i.e. 150 mg/kg.

Each group was consisted of two or three rats, and each test liquid was administered by using oral probe.

After 15, 30, 60 and 120 minutes from administration of test liquid, celiotomy of rat of each group was carried out under anethesia with ether, and immediately about 5 ml of blood is taken from inferior vena cava. This blood was kept standing for 1 to 2 hours under the temperature atomosphere of 4 ℃ as it is, and then was centrifuged for 10 minutes under the same temperature atomosphere to separate a serum.

The nucleic acid base concentration in the serum thus obtained was measeured in accordance with the following method, and the mesuring value was estimated as oral absorption amount of nucleic acid base.

Method for measuring nucleic acid base concentration in serum

The serum obtained by the above procedure was analyzed by high performance liquid chromatography, after pre-treating as mentioned below.

That is, in the case of determination of uracil and thymine, 5 ml of ethyl acetate was added to 1 ml of serum mentioned above, followed by shaking under acidic condition in the presence of hydrochloric acid. Then, ethyl acetate layer was separated, was heated and evaporated to dryness at 40℃, and was dissolved into a small amount of water. 10 μl of a pre-treated sample thus obtained was applied to "ULTRON N-$C_{18}$-L column " (5 μm, 4.6 ID mm × 150mm) manufactured by SHINWA KAKOU SHA, 5 mM tetrabutyl anmonium solution (pH 5 to 6) containing 2 % methanol was flowed through the coloumn as a mobile phase, and analysis was carried out at 1 ml/minute of flow rate and 260 nm of detection wave length.

Also, cytosine and adenine were determined as mentioned below. 200 to 400 μl of the serum containing these nucleic acid base was mounted onto "Ultrafree C3GC cenrifugal filtration tube" (manufactured by MILLIPORE compony) followed by centrifuging at 10000 rpm for 10 minutes. Then, 20 μl of the obtained filtrate was applied to "CHEMCOSORB 300-$C_{18}$ column"(5 μm, 4.6 ID mm × 150mm) manufactured by CHEMCO compony, 20 mM phosphate buffer solution (pH 3.8) was flowed through the coloumn as a mobile phase, and analysis was carried out at 1 ml/minute of flow rate and 260 nm of detection wave length.

Respective concentration of nucleic acid base was caluculated from chromatogram obtained by analyzing samples having a known concentration, which were prepared by adding standard nucleic acid base to normal rat serum, under the same conditions as mentioned above.

Figs 1 to 4 show the test results in the case that L-cysteine was used as test compound, i.e. effective ingredient compound of this invention. In each graph, used nucleic acid base is adenine (Fig. 1), thymine (Fig.2), uracil (Fig. 3) and cytosine (Fig. 4). The axis of ordinate and the axis of abscissa indicate nucleic acid base concentration ($\mu$g/ml) in blood and time (min.) after orally administering test liquid, respectively.

Furthermore, by using hypoxanthine as nucleic acid base, absorption inhibiting effect of hypoxanthine in virtue of the effective ingredient compound of this invention was examined in accordance with the following method.

[8 - $^{14}$C]hypoxanthine (49.0 mCi/m mol, 250 $\mu$Ci/ml) was added and blended to both of test liquid for control group, containing 5 mg/ml of hypoxanthine and another test liquid for experimental group, which was prepared by adding 15 mg/ml of L-cysteine to the test liquid of the control group, so that concentration of hypoxanthine is 10 $\mu$Ci/ml. Each test liquid was orally administered to rats at dose of 1ml per 100 g of rat weight by oral probe. Afer 15, 30 and 60 minutes from administration, blood was taken out from caudal vein of rat of each group, and blood plasma was separated by centrifugation.

For the purpose of deproteination, 1 ml of ice-cooled methanol was added to 0.1 ml of the plasma thus obtained, followed by centrifuging at 3000 rpm for 10 minutes. 0.5 ml of supernatant was contained into a vial, and 10 ml of liquid scintillator (AQUASOL - II) was added to measure the radioactivity with liquid scintillation counter.

From the radioactivity obtaind, the amount of absorbed hypoxanthine was estimated as the corresponding amount of[8 - $^{14}$C]hypoxanthine included in 1 ml of blood. Test result is shown in graph of Fig. 5. In this Fig. 5, the axis of ordinate indicates the corresponding amount of $^{14}$C - hypoxanthine in blood, and the axis of abscissa indicates time (min.) after oral administration of test liquid. Also, (1) and (2) in graph indicate experimental group (using the test liquid including L-cysteine) and control group (using the test liquid which does not include L-cysteine), respectively. Each value is the mean for three rats.

Also, by using L-methionine as a test compound and uracil as nucleic acid base, the test was carried out in the same manner as mentioned above. The test result is shown in Fig. 6 by the same manner as the other drawings mentioned above.

In each graph, (1) and (2) in graph indicate experimental group (using the test liquid including the effective ingredient compound) and control group (using the test liquid which does not include the effective ingredient compound), respectively. Each value is the mean for two to three tats.

From Figs. 1 to 6, it is indicated that effective ingredient compound of this invention lowers concentration of nucleic acid base in serum significantly.

What is claimed is:

1. A composition for inhibiting absorption of nucleic acid base, which is taken into a living body to inhibit absorption of nucleic acid base in the living body, containing, as an effective ingredient, thio-lower alkanoic acid or salt thereof represented by the general formula:

$$R^1 - S - R^2 - COOH$$

wherein $R^1$ is a hydrogen atom or a lower alkyl group, and $R^2$ is a lower alkylene group which may have an amino group.

2. The composition for inhibiting absorption of nucleic acid base according to claim 1, wherein $R^1$ is a hydrogen atom and $R^2$ is a lower alkylene group having an amino group.

3. The composition for inhibiting absorption of nucleic acid base according to claim 1, wherein $R^1$ is a lower alkyl group and $R^2$ is a lower alkylene group having an amino group.

4. The composition for inhibiting absorption of nucleic acid base according to claim 1, wherein said thio-lower alkanoic acid is L-cysteine.

5. The composition for inhibiting absorption of nucleic acid base according to claim 1, wherein said thio-lower alkanoic acid is L-methionine.

6. The composition for inhibiting absorption of nucleic acid base according to claim 1, wherein the composition is prepared to the orally taken form.

7. The composition for inhibiting absorption of nucleic acid base according to claim 6, wherein $R^1$ is a hydrogen atom and $R^2$ is a lower alkylene group having an amino group.

8. The composition for inhibiting absorption of nucleic acid base according to claim 6, wherein $R^1$ is a lower alkyl group and $R^2$ is a lower alkylene group having an amino group.

9. The composition for inhibiting absorption of nucleic acid base according to claim 6, wherein said thio-lower alkanoic acid is L-cysteine.

10. The composition for inhibiting absorption of nucleic acid base according to claim 6, wherein said thio-lower alkanoic acid is L-methionine.

Fig. 1

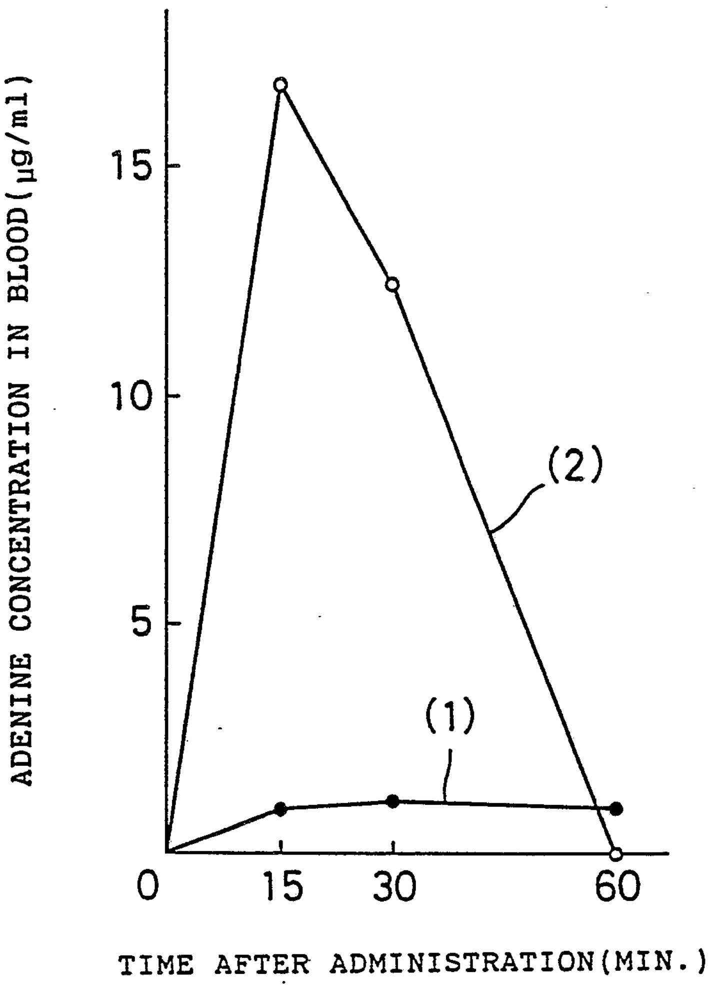
ADENINE CONCENTRATION IN BLOOD (μg/ml)
15
10
5
0
15
30
60
(1)
(2)
TIME AFTER ADMINISTRATION(MIN.)

Fig. 2

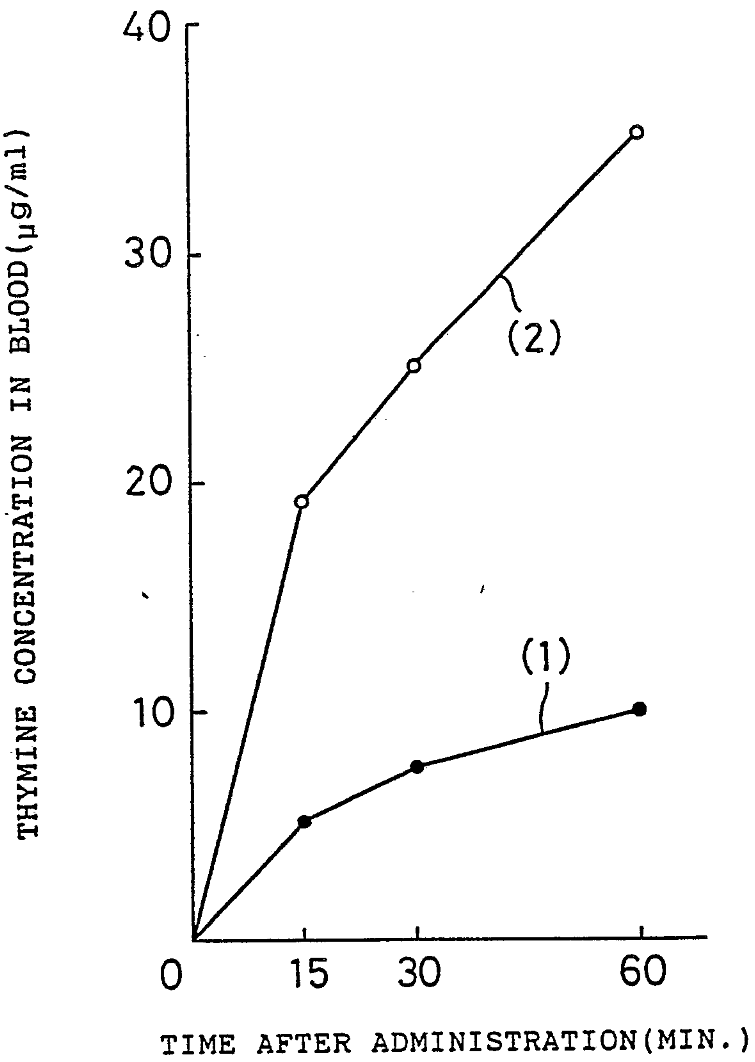
THYMINE CONCENTRATION IN BLOOD (μg/ml)
40
30
20
10
(2)
(1)
0
15
30
60
TIME AFTER ADMINISTRATION (MIN.)

Fig. 3

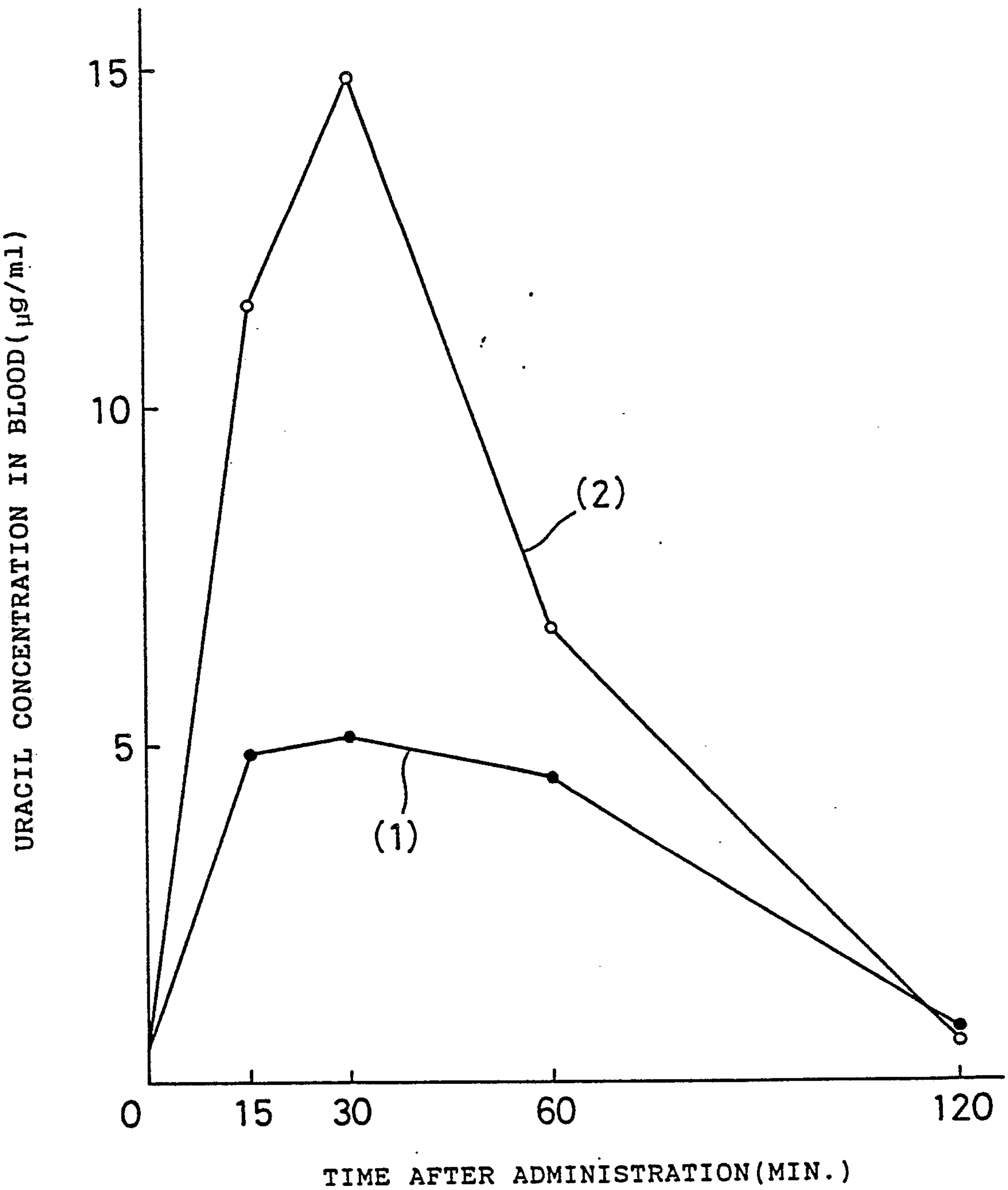
15
10
5
0
15
30
60
120
(1)
(2)
URACIL CONCENTRATION IN BLOOD(μg/ml)
TIME AFTER ADMINISTRATION(MIN.)

Fig. 4

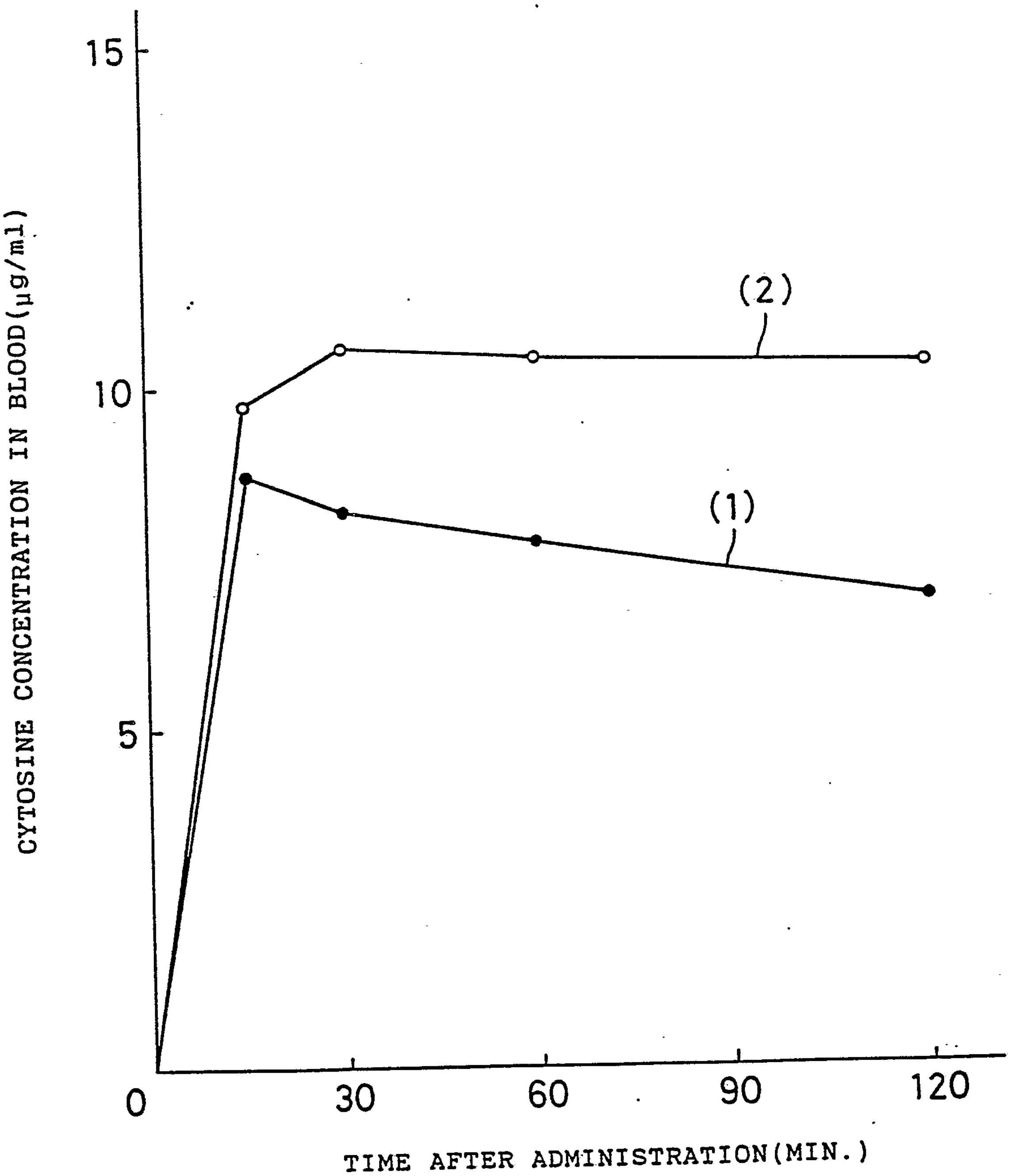
15
10
5
0
30
60
90
120
(1)
(2)
CYTOSINE CONCENTRATION IN BLOOD (μg/ml)
TIME AFTER ADMINISTRATION (MIN.)

Fig. 5

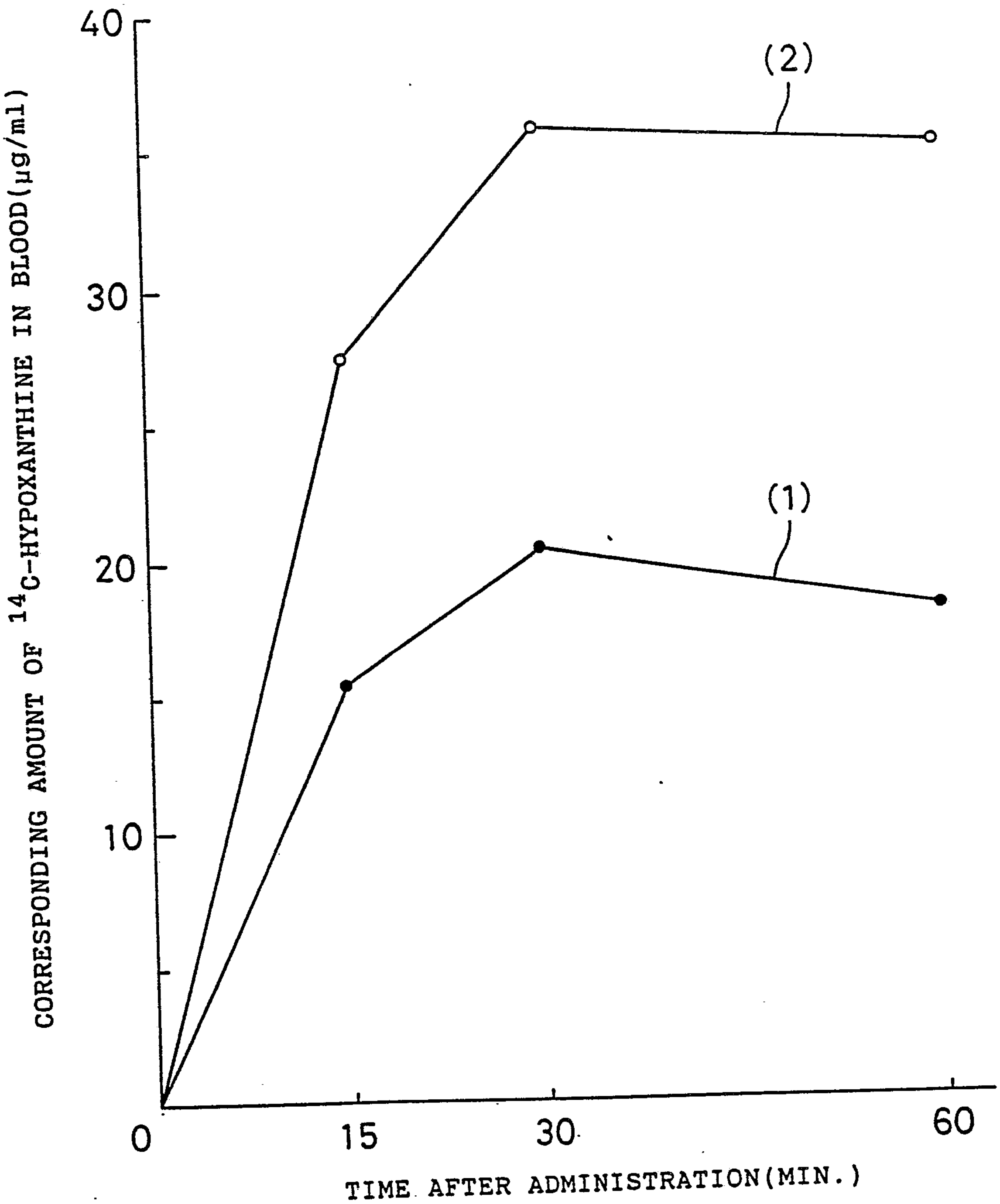
40
30
20
10
0
15
30
60
(2)
(1)
CORRESPONDING AMOUNT OF $^{14}$C-HYPOXANTHINE IN BLOOD(μg/ml)
TIME AFTER ADMINISTRATION(MIN.)

Fig. 6

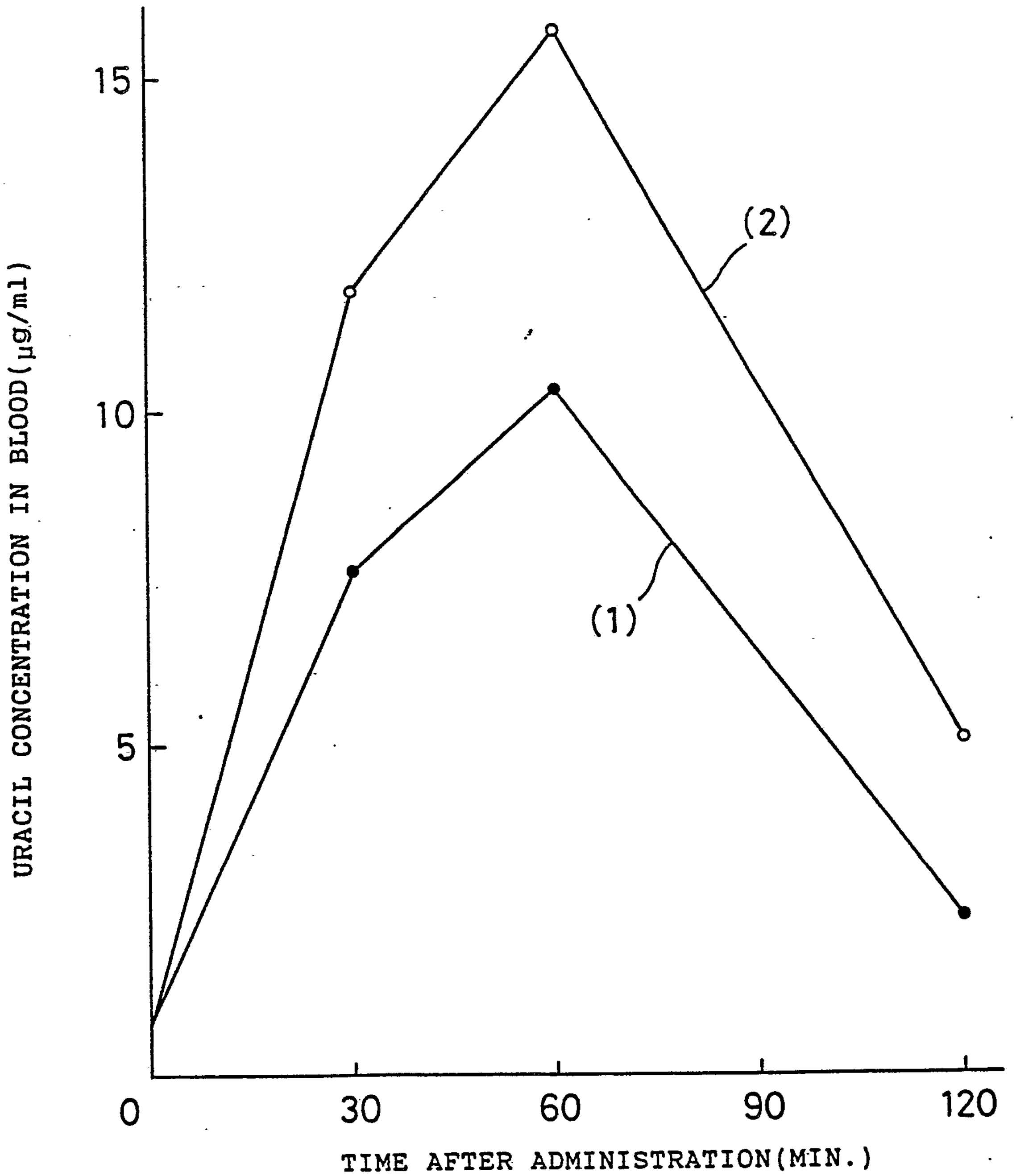
15
10
5
0
30
60
90
120
(1)
(2)
URACIL CONCENTRATION IN BLOOD (μg/ml)
TIME AFTER ADMINISTRATION (MIN.)

# INTERNATIONAL SEARCH REPORT

International Application No PCT/JP89/00313

**I. CLASSIFICATION OF SUBJECT MATTER** (if several classification symbols apply, indicate all) [8]

According to International Patent Classification (IPC) or to both National Classification and IPC

Int. $Cl^4$ A61K31/19// A23L1/03

**II. FIELDS SEARCHED**

Minimum Documentation Searched [7]

| Classification System | Classification Symbols |
|---|---|
| IPC | A61K31/19, A23L1/03 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | Chemical Abstracts, Vol. 102, No. 7, Abstract No. 102(7):61163b, 1985, (Columbus, Ohio, U.S.A.) Amubode, F.O., et. al., J. Agric. Sci., 103(3), 497-502 | 1-10 |
| A | Chemical Abstracts, Vol. 95, No. 17, Abstract No. 95(17):148998p, 1981 (Columbus, Ohio, U.S.A.) Ueda, H., et. al., Nutr. Rep. Int., 24(1), 85-94 | 1-10 |
| A | Chemical Abstracts, Vol. 95, No. 5, Abstract No. 95(5):41169s, 1981, (Columbus, Ohio, U.S.A.) Aguilar, F., et. al., Bol. Soc. Quim. Peru. 45(3),256-65 | 1-10 |
| A | Chemical Abstracts, Vol. 91, No. 9, Abstract No. 91(9):73452n, 1979, (Columbus, Ohio, U.S.A.) Muramatsu, T., et. al., J. Nutr., | 1-10 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| June 12, 1989 (12. 06. 89) | June 26, 1989 (26. 06. 89) |
| **International Searching Authority** | **Signature of Authorized Officer** |
| Japanese Patent Office | |

**FURTHER INFORMATION CONTINUED FROM THE SECOND SHEET**

| | | |
|---|---|---|
| | 109(6), 1057-62 | |
| A | Chemical Abstracts, Vol. 81, No. 23, Abstract No. 81(23):150847t, 1974, (Columbus, Ohio, U.S.A.) Kal'nitskii, B.D., Byull. Vses. Nauchno-Issled. Inst. Fiziol., Biokhim. Pitan. Skh. Zhivotn., 7(2), 20-2 | 1-10 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers ........, because they relate to subject matter not required to be searched by this Authority, namely:

2.☐ Claim numbers ........, because they relate to parts of the international application that do not comply with the prescribed requirements to such an extent that no meaningful international search can be carried out, specifically:

3.☐ Claim numbers ........, because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.